# EUROPEAN PATENT APPLICATION

(11) **EP 0 572 907 A2**
(43) Date of publication of application: **08.12.1993**
(21) Application number: 93108466.9
(22) Date of filing: 26.05.1993
(51) Int. Cl.: C12P 19/34, C07H 1/08, C01B 33/113

(54) **Chemically synthesized silanes that bind nucleic acids**

(30) Priority: 01.06.1992 US 891065
(71) Applicant: Becton Dickinson and Company, Franklin Lakes, New Jersey 07417-1880 (US)
(72) Inventor: Woodard, Daniel L., Raleigh, North Carolina 27613 (US)
(74) Representative: Gerbino, Angelo

(57) **Abstract**

Silane compounds including silicone polymers and silicon tetrols useful for separation, isolation and recovery of nucleic acids from heterogeneous mixtures and methods employing the compounds.

## Description

### FIELD OF THE INVENTION

The present invention relates to reagents for binding nucleic acids, and in particular to solid silanol compounds useful as reagents for recovering, purifying or isolating nucleic acids from heterogeneous mixtures containing other, non-nucleic acid molecules.

### BACKGROUND OF THE INVENTION

Certain silicon-containing compounds are known to bind nucleic acids and have been used for the purification and/or isolation of nucleic acids from heterogeneous mixtures. Best known for purification of nucleic acids are silica or glass particles (silicon dioxide) or the fossilized cell walls of unicellular algae (diatoms, also containing silicon dioxide). Methods for isolation of nucleic acids using particulate silica-containing binding agents are described by Marko, et al. (1982. Anal. Biochem. 121: 382-387), Boom, et al. (1990. J. Clin. Microbiol. 28: 495-503; European Patent Application 90292006/39) and Yamada, et al. (1990. J. Virol. Mtds. 27: 203-210). Nucleic acids have also been recovered from heterogeneous mixtures by passing the mixtures through glass fiber filters (Chen, et al. 1980. Anal. Biochem. 101: 339-341; Chow, et al. 1989. Anal. Biochem. 183: 42-45). McCormick (1989. Anal. Biochem. 181: 66-74) has described silica gel particles derivatized with phenol and their use in removal of proteins from nucleic acid samples by solid phase extraction procedures, thereby leaving the purified nucleic acid in solution.

The Prep-A-Gene™ DNA purification matrix (aluminum silicate diatoms) is commercially available from Bio-Rad Laboratories (Richmond, California) and has been described by Willis, et al. (1990. Biotechniques 9: 92-99). This matrix has a DNA binding capacity of 0.2 µg supercoiled DNA/µL of matrix. Linear and supercoiled DNA of about 0.2 - 20 Kb can be purified or concentrated using the Prep-A-Gene™ matrix. For use with the purification matrix, the manufacturer supplies a perchlorate binding buffer (50 mM Tris, 1 mM EDTA, 6 M NaClO₄, pH 7.5), a wash buffer (40 mM Tris, 4 mM EDTA, 0.8 M NaCl, pH 7.4, in approximately 45-50% ethanol) and an elution buffer (10 mM Tris, 1 mM EDTA, pH 8.0).

The silanol compounds of the present invention provide superior binding and recovery of nucleic acids as compared to commercially available products comprising silicon dioxide, such as the Prep-A-Gene™ matrix.

### SUMMARY OF THE INVENTION

The silanol compounds of the invention include both polymeric reagents (silicone diol polymers) and nonpolymeric silanol reagents (silicon tetrols) either alone or in mixtures with silane polymers. As the compounds of the invention are solids, separation of the nucleic acids after binding is easily accomplished by centrifugation, filtration and the like. The bound nucleic acids may then be eluted from the binding reagent and recovered by treatment of the solid with dilute buffers and, optionally, heating.

The silicone diol reagents of the invention are polymers of the following monomeric structure: 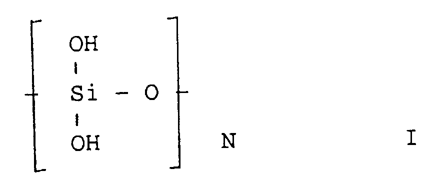
wherein N represents the number of repeating units of the monomer. For convenience, hereinafter the silicone diol polymers of the invention will be referred to as Compound I or abbreviated (SiO₃H₂)_{N}.

Compound I nucleic acid binding reagents are synthesized by protonation of a sodium meta silicate polymer according to the following formula:

### Reaction A

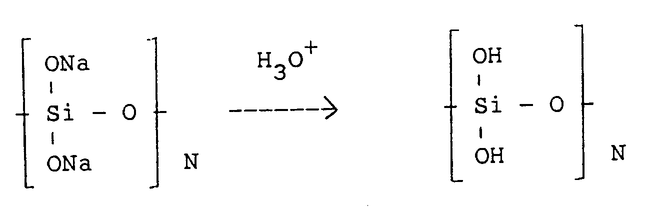

Other nucleic acid binding reagents of the invention have the following structure: 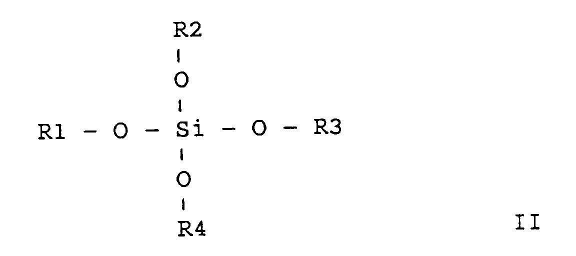
wherein R1, R2, R3 and R4 may each be hydrogen or one or more additional monomeric unit(s) linked to the oxygen through the silicon moiety to form a silane polymer. When R1, R2, R3 and R4 are all hydrogen, the compound is a silicon tetrol.

Compound II is synthesized by hydrolysis of silicon tetrachloride as follows:

### Reaction B

SiCl₄ + 4 H₂O ------> Si(OH)₄ + 4 HCl

While not wishing to be bound by any particular theory, Applicant believes that Reaction B may produce a nucleic acid binding reagent which is a mixture of silicon tetrol and a variety of silane polymers representing variable degrees of polymerization of the monomeric subunit with additional monomers. Because the Reaction B reaction product has not been extensively characterized and quantitated, for convenience the nucleic acid binding reagent produced by the foregoing chemical reaction will hereinafter be referred to as Compound II or abbreviated Si(OH)₄. The term "Compound II" and the abbreviation "Si(OH)₄" are both intended to encompass the individual or mixed products of Reaction B, including the silicon tetrol and/or polymeric derivatives thereof as described above.

### DESCRIPTION OF THE DRAWINGS

Fig. 1 A is a graph comparing the DNA binding capacities of (SiO₃H₂)_{N}, Si(OH)₄ and the Prep-A-Gene™ matrix for high molecular weight lambda DNA.

Fig. 1 B is a graph comparing the DNA binding capacities of (SiO₃H₂)_{N}, Si(OH)₄ and the Prep-A-Gene™ matrix for low molecular weight lambda DNA.

Fig. 2 A is a graph comparing the DNA binding capacities of (SiO₃H₂)_{N}, Si(OH)₄ and the Prep-A-Gene™ matrix for high molecular weight lambda DNA without heating.

Fig. 2 B is a graph comparing the DNA binding capacities of (SiO₃H₂)_{N}, Si(OH)₄ and the Prep-A-Gene™ matrix for middle molecular weight lambda DNA without heating.

Fig. 2 C is a graph comparing the DNA binding capacities of (SiO₃H₂)_{N}, Si(OH)₄ and the Prep-A-Gene™ matrix for low molecular weight lambda DNA without heating.

### DETAILED DESCRIPTION OF THE INVENTION

New chemical reagents which bind nucleic acids in solution have been developed. They are useful for separation of nucleic acids from heterogeneous mixtures which contain non-nucleic acid molecules such as proteins and other cellular components. After binding to the reagents, the nucleic acids can be separated from the solution along with the solid reagent by centrifugation, filtration and the like. The nucleic acids remain bound to the reagent through subsequent washing steps and can be eluted therefrom in essentially pure form by exposure to dilute buffers and, optionally, increased temperatures. The purified nucleic acids are recovered in a form which is suitable for use in many subsequent procedures and reactions, including polymerase chain reactions, molecular cloning, restriction endonuclease digestion, reverse transcription, and use as molecular probes.

Both the (SiO₃H₂)_{N} and Si(OH)₄ nucleic acid binding reagents are solids and bind nucleic acids efficiently. While not wishing to be bound by any particular theory of how the invention operates to provide the advantages hereinafter described, Applicant hypothesizes that the relatively high number of silanol moieties in the chemical structure of the reagents provides a hydrophilic surface which facilitates and improves binding of negatively charged nucleic acids. The reagents may therefore be used to bind and recover DNA or RNA in either single stranded or double stranded form. Because the reagents are solids, separation of bound nucleic acids from the remainder of a heterogeneous solution is easily accomplished by centrifuging or filtering the solid out of the solution. Alternatively, the reagents may be used as a column chromatography medium or packed into a filtering device. In one embodiment the reagents may be packed into a filtering device which attaches to a syringe. Using such a device the heterogenous solution can be forced through the filtering device using the syringe, allowing binding of nucleic acids while other unwanted components pass through. This provides a simplified, rapid nucleic acid purification procedure. These and other methods suitable for purification and/or isolation of nucleic acids using the inventive reagents are described in the background publications referenced above.

The Compound I nucleic acid binding reagents of the invention are silicone diol polymers comprising two or more monomeric units according to the following structure: 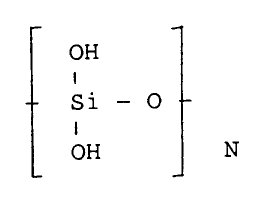
wherein N represents the number of repeating units in the polymeric structure.

The silicone diol polymer reagents may be synthesized by protonating a sodium metasilicate polymer by treatment with a strong acid such as sulfuric acid, HCl or glacial acetic acid. The length (i.e., "N") of commercially available sodium metasilicate polymers is not known and Applicant believes that they are likely to be mixtures of different lengths. The present disclosure describes synthesis of the nucleic acid binding reagents of the invention using these commercially available sodium metasilicate polymers. However, if sodium metasilicate polymers of homogeneous length become available, these may be substituted in the syntheses disclosed herein and the corresponding reaction products are intended to be included within the scope of the invention.

In the preferred Compound I synthetic procedures the sodium metasilicate polymer is mixed with 20% sulfuric acid and the reaction is allowed to proceed overnight. The product is then filtered, washed with water, and dried to a particulate form such as a powder or grains. If desired, the particulate may be stored in a dessicator.

The Compound II nucleic acid binding reagents have the following general structure: 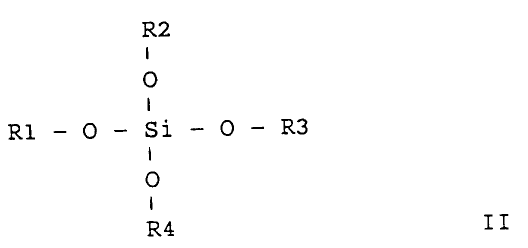
wherein R1, R2, R3 and R4 may each be hydrogen or one or more additional Compound II moieties linked to the oxygen through the silicon residue. The Compound II nucleic acid binding reagents therefore include monomeric reagents in which R1, R2, R3 and R4 are hydrogen: 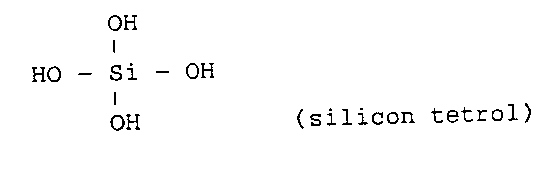
as well as related silane polymers in which one or more hydrogens of the tetrol are replaced with one or more additional monomeric units. Examples include but are not limited to: 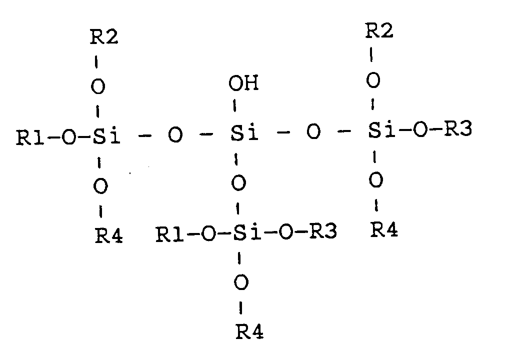
in which R1, R3 and R4 are additional monomeric units and R2 is hydrogen. Alternatively, all "R" groups may be monomeric units of Compound II or only one or two "R" groups may be monomeric units with the rest being hydrogen. Of course, the "R" groups on the additional monomeric units may in turn be either hydrogen or further monomeric units, resulting in the formation of polymeric molecules of varying size and structural conformation.

The Compound II nucleic acid binding reagents of the invention may be synthesized by hydrolysis of silicon tetrachloride (SiCl₄). In the preferred synthesis, SiCl₄ is added dropwise to water until a gel forms. The gel is air dried and oven dried to produce a powder. The product produced by this reaction has not been extensively characterized but, without being bound by this theory, Applicant believes that the hydrolysis of SiCl₄ may produce a mixture of reaction products which include the silicon tetrol and a variety of silane polymers representing the various combinations of R1, R2, R3 and R4 substitutions (i.e., hydrogen or polymerized monomeric subunits at each position). Based upon the working hypothesis that the silanol moieties of the reagents bind to nucleic acids, Applicant believes that each of these reaction products individually or in combination with one or more reaction products would be useful for isolation and/or purification of nucleic acids as well. Therefore, the substantially homogeneous individual reaction products of the hydrolysis of silicon tetrachloride are also intended to be included in the invention.

It has also unexpectedly been found that certain reagents with a large number of available hydroxyl groups can be synthesized, but that nucleic acids may be bound so tightly that elution and recovery of the nucleic acids is difficult or impossible using conventional methods. One such compound is synthesized by reaction of silicon tetrachloride with sodium metasilicate and treatment of the reaction product with sodium hydroxide. This reagent is a polymer having the structure: 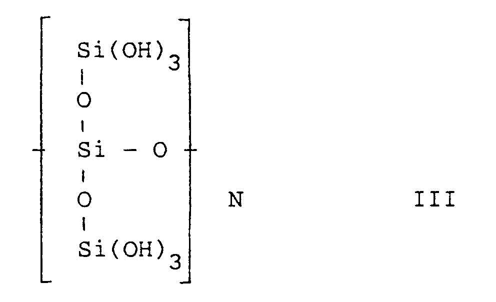
Although Compound III is not generally suitable for purification and/or isolation of nucleic acids, its unexpected ability to essentially irreversibly bind nucleic acids makes it useful for removal of nucleic acids from heterogeneous mixtures in which such nucleic acids are considered an undesirable contaminant. Reagents such as Compound III are therefore useful in the purification and/or isolation of non-nucleic acid molecules which exist in a heterogeneous mixture including nucleic acids.

For all of the nucleic acid binding reagents, smaller particles are preferred for their larger surface area and increased nucleic acid binding capacity. The powders produced by the syntheses described above may therefore be sized prior to use by filtering through a mesh with a desired porosity to select particles of a more uniform desired size.

Isolation and recovery of nucleic acids from heterogeneous solutions or removal of contaminating nucleic acids using the reagents of the invention may be performed by mixing the reagent with the solution and incubating the mixture for a sufficient period of time to allow binding of the nucleic acids to the reagent. Preferably, the binding step is performed in the presence of a suitable buffer. More preferably the binding buffer is 50 mM Tris, 1 mM EDTA and 6 M perchlorate (NaClO₄), pH 7.5.

When isolation or purification of the nucleic acids is desired, the reagents with the bound nucleic acids are recovered from the mixture and the nucleic acids are eluted from the reagents. When removal of nucleic acids is desired, the reagents with the bound nucleic acids are separated and purification of the desired component from the solution is continued. The incubation of reagents with the heterogeneous solution may be performed at room temperature or with heating, depending on whether binding of double stranded or single stranded nucleic acids is desired. Including chaotropes in the binding reaction also facilitates binding of single stranded nucleic acids to the reagents. Suitable chaotropic agents include, but are not limited to, 6 M NaClO₄, 6 M guanidine HCl and 6 M guanidine thiocyanate.

For most applications, recovery of the reagents with the bound nucleic acids from the solution is accomplished by centrifugation or filtration. However, alternative methods will be apparent to the practitioner and may easily be adapted to specific needs without the exercise of inventive skill. These include, but are not limited to, isolation of nucleic acids by column chromatography using the inventive reagents as an affinity matrix and recovering bound nucleic acids by elution from the column with dilute buffers. Column chromatography using the inventive reagents may also be used to remove nucleic acids from a solution by passing the solution through the column and collecting the flow-through for further processing. Alternatively, the reagents may be prepared in the form of a filtering device through which the heterogeneous solution is forced by pressure or drawn by vaccuum, recovering the bound nucleic acids if desired by washing the device with dilute buffers.

Elution of the bound nucleic acids is preferably accomplished by washing the reagents with water or a dilute Tris buffer. When Tris buffers are used for elution, the concentration of Tris is preferably between 1 mM and 20 mM, more preferably about 10 mM. The most preferred elution buffer is 10 mM Tris, 1 mM EDTA, pH 8.0. The buffers supplied with the Prep-A-Gene nucleic acid recovery kit are also useful in the invention for both binding and elution of nucleic acids according to the manufacturer's instructions.

Once eluted from the binding reagents, the nucleic acids may be recovered from the elution buffer and/or concentrated using any of the means known in the art. For example, total DNA or RNA may be precipitated from the buffer by addition of ethanol. Alternatively, specific nucleic acid species may be recovered and/or concentrated by application to an agarose or polyacrylamide gel for electrophoresis, and a desired band eluted from the gel in a defined volume of liquid. Specific species of DNA or RNA may also be recovered and isolated from the elution buffer by molecular cloning. The foregoing procedures are well known in the art. See, for example, Molecular Cloning, T. Maniatis, Cold Spring Harbor Laboratories. The preferred method for recovery of nucleic acids from the elution buffer will be dependent upon the results desired and the purpose for which the nucleic acids are isolated.

Nucleic acids isolated by the above procedures using the nucleic acid binding reagents of the invention are recovered in a form which is suitable for use in many molecular genetics procedures without further manipulation or purification. For example, recovered DNA or RNA may be amplified by the polymerase chain reaction (PCR) or modifications of PCR. The RNA may be used as templates for synthesis of cDNA or single stranded DNA may be used as template for synthesis of a second, complementary DNA strand. The recovered nucleic acids may also be cloned into suitable cloning vectors by addition of homopolymer tails, addition of defined linkers, or digestion with restriction endonucleases appropriate for the cloning site in the vector. The recovered nucleic acids may further be used as molecular probes for Southern blots, Northern blots, in situ hybridizations and hybridization screening of recombinant clones.

Certain features of the invention are illustrated in the following experimental Examples. These Examples are not intended to limit the invention as defined by the appended claims, as modifications and variations will be apparent to those skilled in the art without the exercise of inventive skill upon reading the instant specification. Such modifications and variations are included within the scope of the invention.

### EXAMPLE 1

Compound I, (SiO₃H₂)_{N}, was synthesized as follows. Sodium meta silicate polymer (SiO₃Na₂)_{N}, 2 g - Petrarch, Bristol, Pennsylvania) was mixed with 50 mL of 20% H₂SO₄ in a 125 mL erlenmeyer flask and stored overnight. The product was filtered, washed ten times with 20 mL of water and air dried for 20 min. This product was then oven dried to a powder and stored in a dessicator until use.

Compound II, Si(OH)₄, was synthesized as follows. SiCl₄ was added dropwise to 25 mL of H₂O in a 250 mL beaker until a gel formed. The reaction produced HCl gas. The gel was washed three times with 10 mL of water followed by three washes with 10 mL of acetone. It was first air dried and then oven dried until a white powder formed.

Infrared analysis of the reaction products showed a high concentration of silanols for Compound I and an even higher concentration of silanols for Compound II. Compound I also appeared to be more defined in the Si-O-Si region of the spectrum.

### EXAMPLE 2

The DNA binding and recovery properties of the reagents prepared above were compared to SiO₂, the commercially available Prep-A-Gene™ DNA binding matrix. This product has been considered the best available DNA binding surface. Five different concentrations of HindIII digested lambda phage DNA were tested for each matrix/reagent - 2 µL (about 1.3 µg), 1 µL (about 0.65 µg), .5 µL (about 0.3 µg), .25 µL (about 0.15 µg) and .125 µL (about 0.075 µg) in a total volume of 250 µL TE buffer. The test reagents were prepared as stock solutions of 100 mg of solid in 200 µL TE buffer.

To each DNA sample was added either the Prep-A-Gene™ matrix or 30 µL of one of the test reagents and 750 µL of Prep-A-Gene binding buffer. The samples were shaken at room temperature for 5 min. and heated at 60^{o} C for 10 min. The samples were centrifuged to pellet the matrices/reagents, the liquid was decanted and the binding step was repeated. After a second centrifugation and decanting, the matrices/reagents were washed with 500 µL of Prep-A-Gene™ wash buffer, mixed 10 min. at room temperature, centrifuged and decanted. Residual ethanol was removed by treatment at 60^{o} for 10 min. Twenty-five µL of Prep-A-Gene™ elution buffer was added and the samples were again heated at 60^{o}C for 10 min. After centrifugation the elution buffer was collected and the elution step was repeated. The elution buffer collected from the second elution step was combined with the buffer collected from the first elution.

The DNA recovered from each procedure was analyzed on a 1% agarose gel run in 1X TAE and stained with ethidium bromide (7 µL of elution buffer + 3 µL of loading dye). Standards containing the amounts of lambda DNA tested for recovery were applied to the gels as well. To quantitate the DNA recovered by each procedure, negatives of gel photographs were scanned with a Gelman Sciences ACD-18 Automatic Computing Densitometer, scanning 65 mm, O.D. range 0.25, slit 0.2 X 10 mm, light wave 525 nm.

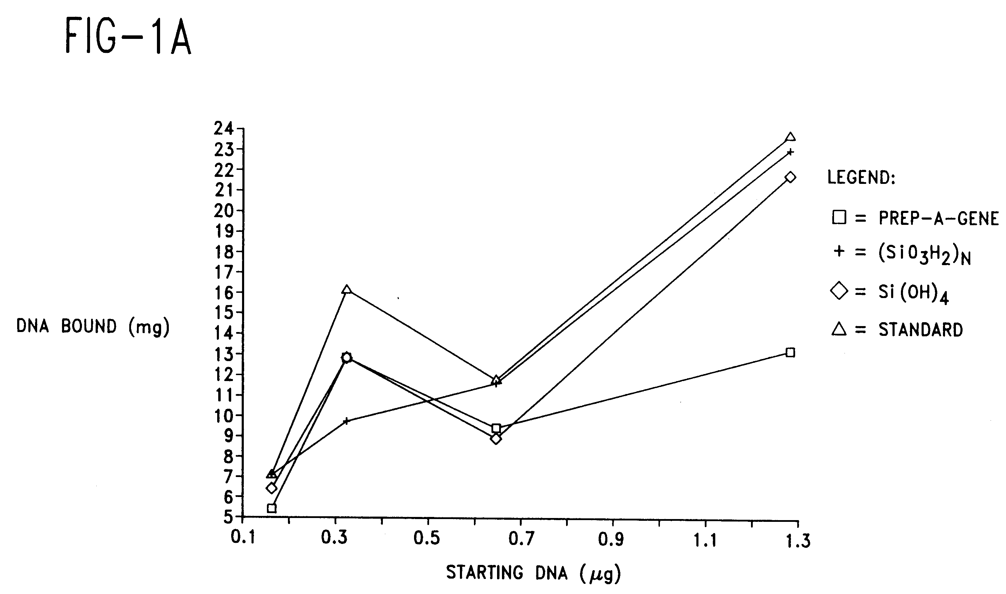

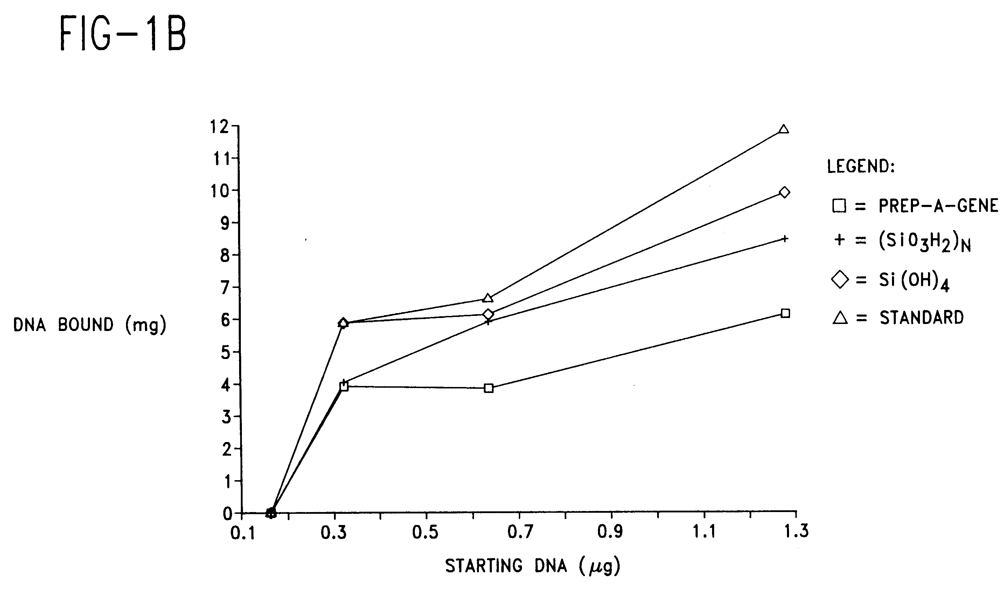

A plot of the results of the densitometer scans for high molecular weight bands of lambda DNA is shown in Fig. 1 A. All of the inventive reagents tested exhibited superior recovery of nucleic acids as compared to the commercially available Prep-A-Gene™, approaching quantitative recovery of added nucleic acid when compared to the standard curve. The inventive nucleic acid binding reagents were also superior to Prep-A-Gene™ for recovery of low molecular weight lambda DNA (Fig. 1 B).

Although pipetting errors were evident at low volumes for all scans, the error was consistent for all matrices/reagents tested and therefore does not affect the interpretation of the results. This experiment demonstrates that the Si(OH)₄ and (SiO₃H₂)_{N} nucleic acid binding reagents are superior to commercially available binding reagents for retention and recovery of both low and high molecular weight DNA.

### EXAMPLE 3

The protocol of Example 2 included heating of the samples during binding and elution of nucleic acids and therefore probably demonstrates binding of single stranded DNA. In this experiment, essentially the same protocol was repeated without the heating steps during binding to determine the extent of binding of double stranded DNA to the reagents as compared to a commerically available DNA binding matrix. The lambda DNA was used in more dilute form to reduce pipetting errors due to small volumes.

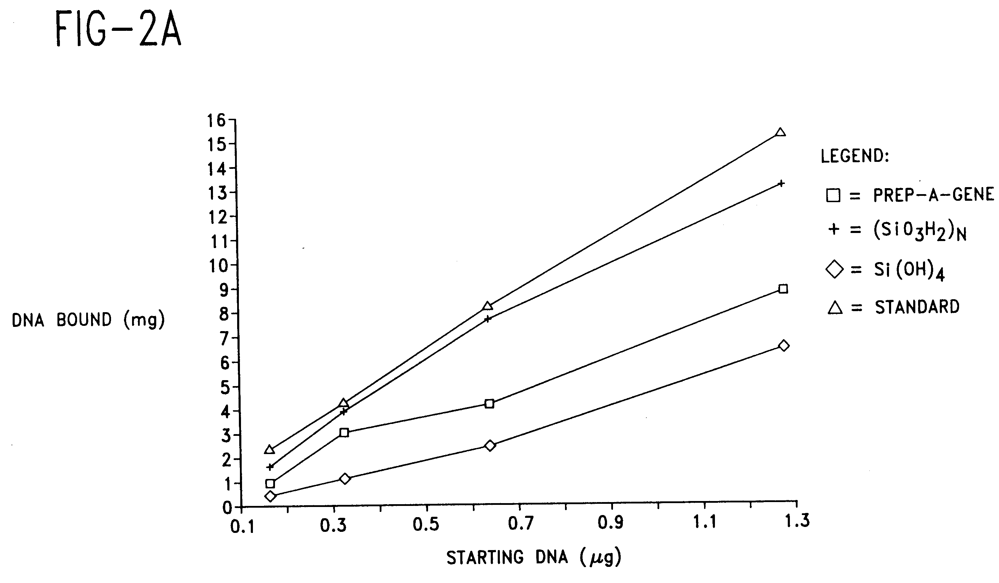

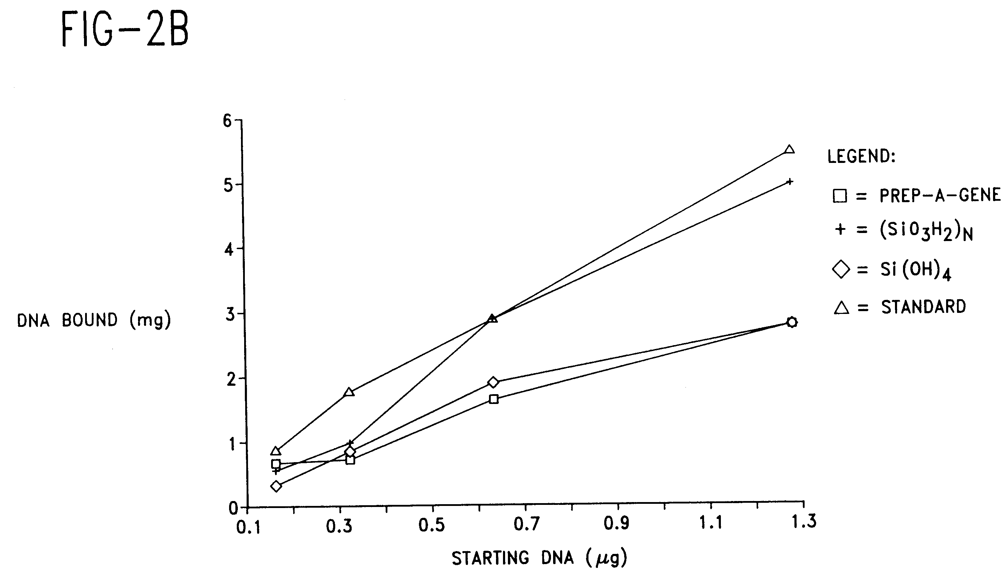

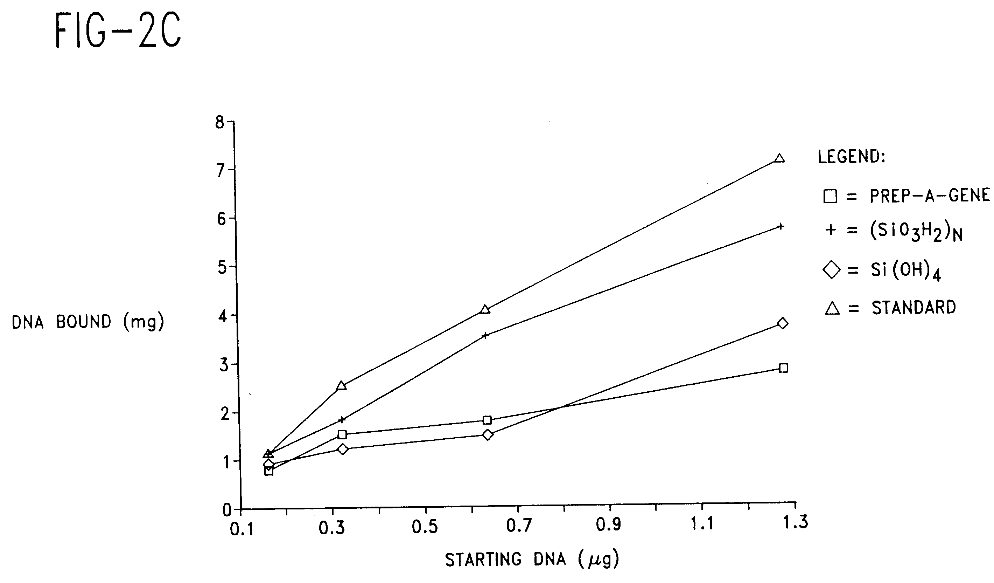

The results of the densitometer scans of the gel photograph negatives are plotted in Figs. 2 A - 2 C. In contrast to the experiment in which heat was applied during the binding step, for each molecular weight of lambda DNA the Si(OH)₄ reagent was equal to or slightly below the Prep-A-Gene™ matrix in DNA retention capacity. In contrast, in the previous experiment the Si(OH)₄ reagent retained more DNA than any of the other binding reagents tested. It is possible that this result is due to a higher degree of hydrogen bonding of Si(OH)₄ with single stranded DNA . However, the (SiO₃H₂)_{N} reagent consistently retained more DNA than Prep-A-Gene™ for all molecular weights and all DNA concentrations tested and in most cases approached quantitative retention of DNA as compared to the standard curve.

## Claims

1. A composition of matter comprising a silicone diol polymer having the structure:

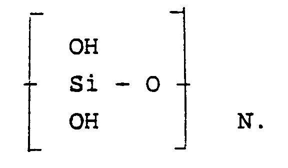

2. A composition of matter comprising a compound having the structure: 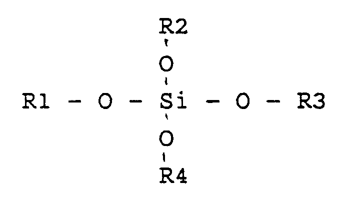 wherein R1, R2, R3 and R4 are each hydrogen or one or more additional moieties of the compound linked to the oxygen through the silicon residue.

3. A composition of matter comprising a silane polymer having the structure:

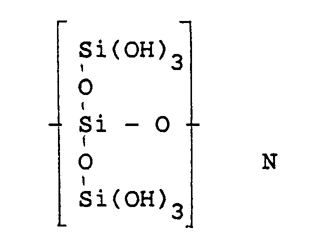

4. A product for binding nucleic acids produced by a process comprising treating sodium metasilicate polymer with strong acid and recovering the product in particulate form.

5. A method for separating nucleic acids from a heterogeneous solution comprising:
a) contacting the heterogeneous solution with a composition of matter comprising a silicone diol polymer having the structure 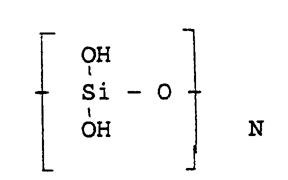 such that the nucleic acids bind to the polymer, and;
b) separating the polymer With the bound nucleic acids from the solution.

6. The method of Claim 5 further comprising isolating the nucleic acids by eluting the bound nucleic acids from the polymer.

7. The method according to Claim 6 wherein the nucleic acids are eluted with heating.

8. A method for separating nucleic acids from a heterogeneous solution comprising:
a) contacting the heterogeneous solution with a composition of matter comprising a compound having the structure 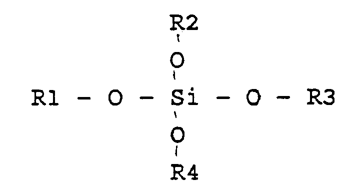 wherein R1, R2, R3 and R4 are each hydrogen or one or more additional moieties of the compound linked to the oxygen through the silicon residue, such that the nucleic acids bind to the composition of matter, and;
b) separating the composition of matter with the bound nucleic acids from the solution.

9. The method of Claim 8 further comprising isolating the bound nucleic acids by eluting the nucleic acids from the composition of matter.

10. The method according to Claim 9 wherein the nucleic acids are eluted with heating.
